(19) Europäisches Patentamt
European Patent Office
Office européen des brevets

(11) Veröffentlichungsnummer: **0 184 694**
**B1**

(12) # EUROPÄISCHE PATENTSCHRIFT

(45) Veröffentlichungstag der Patentschrift: **27.04.88**

(51) Int. Cl.⁴: **C 07 C 121/36**, C 07 C 121/43, C 07 C 120/00, C 01 C 3/10

(21) Anmeldenummer: **85114756.1**

(22) Anmeldetag: **21.11.85**

(54) **Verfahren zur Herstellung von Umsetzungsprodukten des Cyanwasserstoffs.**

(30) Priorität: **29.11.84 DE 3443462**

(43) Veröffentlichungstag der Anmeldung: **18.06.86 Patentblatt 86/25**

(45) Bekanntmachung des Hinweises auf die Patenterteilung: **27.04.88 Patentblatt 88/17**

(84) Benannte Vertragsstaaten: **AT BE CH DE FR GB IT LI NL SE**

(56) Entgegenhaltungen:
**DE-B-1 211 612**
**DE-C-477 437**
**DE-C-669 808**
**US-A-3 742 016**

(73) Patentinhaber: **BASF Aktiengesellschaft, Carl-Bosch- Strasse 38, D-6700 Ludwigshafen (DE)**

(72) Erfinder: **Brunnmueller, Fritz, Dr., Alsenzstrasse 7, D-6703 Limburgerhof (DE)**
Erfinder: **Stecher, Karlheinz, Dr., Osloer Weg 42, D-6700 Ludwigshafen (DE)**
Erfinder: **Kroener, Michael, Dr., Eislebener Weg 8, D-6800 Mannheim 31 (DE)**
Erfinder: **Schneider, Rolf, Dr., Feldbergstrasse 21, D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 184 694 B1

LIBER, STOCKHOLM 1988

**Beschreibung**

Aus der DE-PS 1 211 612 ist die Herstellung von Cyanwasserstoff nach dem sogenannten Formamid-Vakuum-Verfahren bekannt, bei dem man Formamid unter vermindertem Druck verdampft und unter Wärmezufuhr katalytisch in Cyanwasserstoff und Wasser spaltet. In einer unerwünschten Nebenreaktion wird aus Formamid unter den Pyrolysebedingungen in geringen Mengen Ammoniak und Kohlenmonoxid gebildet. Da jedoch Ammoniak die Polymerisation von Cyanwasserstoff katalysiert, ist es bei dem bekannten Verfahren unumgänglich, Ammoniak aus dem Spaltgas durch Waschen mit einer nicht flüchtigen Säure zu entfernen. Das gewaschene Spaltgas wird anschließend auf Atmosphärendruck komprimiert, durch Kühlen weitgehend verflüssigt und gegebenenfalls einer Destillationsanlage zur Aufarbeitung auf reinen Cyanwasserstoff zugeleitet, aus dem dann Umsetzungsprodukte, wie Natriumcyanid oder Acetoncyanhydrin, hergestellt werden, die relativ gefahrlos gehandhabt werden können.

Es ist bekannt, für die Erzeugung des für die Pyrolyse benötigten Unterdrucks Pumpen mit mechanisch bewegten Teilen, z. B. ein- oder mehrstufige Kolbenverdichter oder Kreiselpumpen, zu verwenden. Diese Aggregate haben jedoch den Nachteil, daß die Blausäure darin leicht zu dunkelgefärbten festen Produkten polymerisiert, die die Anlage verstopfen und Anlaß häufiger Betriebsunterbrechungen sind. Verstopfungen durch Polymerisate des Cyanwasserstoffs beobachtet man besonders in den Zylindern und Schieberkästen der Verdichter sowie in den Leitungen und Apparateteilen auf der Druckseite der Verdichter. Gemäß der Lehre der DE-PS 1 211 612 lassen sich die Polymerisatbildungen weitgehend vermeiden, wenn die Temperatur des Cyanwasserstoffhaltigen Spaltgases am Ausgang der Druckseite des Verdichters der Anlage auf eine Temperatur von 55 bis 80, vorzugsweise 60 bis 70° C eingestellt wird.

Gemäß einer anderen bekannten Arbeitsweise soll bei der Cyanwasserstoff-Herstellung keine Polymerisation des Cyanwasserstoffs auftreten, wenn man anstelle der Kolbenverdichter für die Erzeugung des Unterdrucks Dampfstrahlsauger einsetzt. Hierbei ergeben sich jedoch andere Nachteile, so daß sich auch Dampfstrahlsauger in der Technik nicht bewährt haben.

Bei den bekannten Verfahren zur Herstellung von Cyanwasserstoff müssen noch andere Nachteile in Kauf genommen werden. So ist es z. B. notwendig, regelmäßig schmierölhaltige Kondensate am tiefsten Punkt der Anlage abzuziehen, regelmäßige Spülungen der druckseitigen Anlage vorzunehmen oder Stabilisatoren zu verwenden, die die Polymerisation des Cyanwasserstoffs verhindern bzw. zurückdrängen.

Ein grundsätzliches Problem bei der Cyanwasserstoffherstellung besteht jedoch in der Verdichtung sehr großer Mengen an Spaltgas, wozu sehr kostspielige und reparaturanfällige Verdichteraggregate erforderlich sind. So muß man beispielsweise bei einem Druck von 80 mbar und einem stündlichen Formamideinsatz von 900 kg ca. 7750 $m^3$ Gas/h mit Schwefelsäure zur Entfernung von Ammoniak waschen und verdichten. Hinzu kommen noch Trägergas und die aus Nebenreaktionen stammenden Spaltgase wie Kohlenmonoxid, Kohlendioxid und Wasserstoff. Die so auf Atmosphärendruck verdichtete wasserhaltige Blausäure muß kondensiert und destilliert werden und kann erst nach Entfernung von Kohlendioxid mit Natronlauge zu wäßriger Natriumcyanidlösung weiterverarbeitet werden.

Der vorliegenden Erfindung liegt die Aufgabe zugrunde, ein technisch einfacheres Verfahren zur Herstellung von Umsetzungsprodukten des Cyanwasserstoffs im Zusammenhang mit der Herstellung von Cyanwasserstoff durch Pyrolyse zur Verfügung zu stellen. Hierbei wird besonderer Wert auf eine hohe Betriebssicherheit des Verfahrens gelegt.

Die Aufgabe wird erfindungsgemäß mit einem Verfahren zur Herstellung von Umsetzungsprodukten des Cyanwasserstoffs durch Reaktion von Cyanwasserstoff mit Basen oder Carbonylverbindungen dadurch gelöst, daß man durch Pyrolyse bei 250 bis 650° an Feststoffen als Katalysatoren unter einem Druck von 5 bis 200 mbar hergestellten Cyanwasserstoff zusammen mit den anderen Pyrolyseprodukten auf eine Temperatur von 200 bis -10° C kühlt, die höher als Cyanwasserstoff siedenden Produkte kondensiert, das Kondensat aus System ausschleust, den nicht kondensierten Cyanwasserstoff dann-ebenfalls unter verminderten Druck, bei dem auch die Pyrolyse stattfindet - einer Chemiesorption mit Basen oder Carbonylverbindungen bei -20 bis +30° C zuführt und die dabei entstehenden Umsetzungsrodukte des Cyanwasserstoffs aus dem System ausschleust und auf Atmosphärendruck bringt.

Der Cyanwasserstoff wird unter vermindertem Druck durch Pyrolyse von Verbindungen hergestellt, die bei einer Temperatur von 250 bis 650° C Cyanwasserstoff abspalten. Vorzugsweise verwendet man als Cyanwasserstoff-abspaltende Verbindung Formamid. Es können jedoch auch eine ganze Reihe anderer Verbindungen eingesetzt werden. Von Interesse sind hier vor allem N-Acylderivate des 1-Amino-1-cyanethans oder deren Substitutionsprodukte, weil aus diesen Verbindungen neben Cyanwasserstoff andere Stoffe, die z. B. als Monomere geeignet sind, nämlich N-Vinyl-N-Acylamide bzw. die substituierten Produkte hergestellt werden können. Die N-Acylderivate des 1-Amino-1-cyanethans können beispielsweise mit Hilfe der folgenden Formel

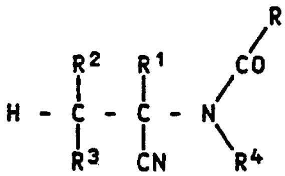

(I),

in der

R = H oder $C_1$- bis $C_6$-Alkyl,

$R^1$, $R^2$, $R^3$ = H, $C_1$- bis $C_6$-Alkyl,

$R^1$ und $R^2$ bzw. $R^2$ und $R^3$ = 5 oder 6 C-Atome enthaltender carbocyclischer Ring und

$R^4$ = H, $C_1$- bis $C_6$-Alkyl

bedeutet, charakterisiert werden. So erhält man beispielsweise aus der Verbindung der Formel

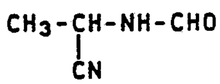

(II)

in einer glatt verlaufenden Reaktion Cyanwasserstoff und N-Vinylformamid ($CH_2$=CH-NH-CHO), aus der Verbindung der Formel

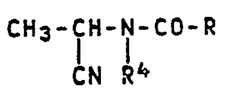

(III)

wenn R = H und $R^4$ = $CH_3$ ist, N-Vinylmethylformamid und HCN und wenn in Formel III R = $CH_3$ und $R^4$ = $CH_3$ bedeutet, N-Vinyl-N-methylacetamid und HCN.

Man kann hier die Entwicklung des Cyanwasserstoffs mit der Herstellung von technisch wertvollen Monomeren koppeln. Die Verbindungen der Formel I und deren Pyrolyse ist aus den deutschen Patentschriften 1 224 304 und 1 228 246 bekannt.

Die Pyrolyse der Cyanwasserstoffabspaltenden Verbindungen erfolgt an Feststoffen. Man kann hierfür diejenigen Feststoffe verwenden, die beispielsweise in der DE-PS 1 224 304 angegeben sind, sowie Alkali- und Erdalkalicarbonate, wie Natriumcarbonat, Kaliumcarbonat, Magnesiumcarbonat, Strontiumcarbonat, Bariumcarbonat, Marmor, Dolomit, Kreide und Magnetit. Als besonders vorteilhaft haben sich solche Katalysatoren erwiesen, die Alkali- oder Erdalkalicarbonate auf α-Aluminiumoxid als Träger enthalten. Vorzugsweise verwendet man Calciumcarbonat, Dolomit oder Mischungen aus Calciumcarbonat und Magnesiumcarbonat auf α-Aluminiumoxid. Katalysatoren dieser Art werden hergestellt, indem man α-Aluminiumoxid mit wasserlöslichen Salzen, z. B. Calciumacetat tränkt und durch eine thermische Behandlung in die entsprechenden Carbonate bzw. Oxide überführt.

Die Pyrolyse wird bei Temperaturen von 250 bis 650, vorzugsweise 300 bis 550° C und unter einem Druck von 5 bis 200, vorzugsweise 10 bis 150 mbar durchgeführt. Die Pyrolyseprodukte, die je nach Bedingungen in der Pyrolysezone mehr oder weniger große Mengen an nicht-umgesetztem Ausgangsmaterial enthalten können, werden unter den in der Pyrolysezone herrschenden Druckbedingungen auf eine Temperatur von 200 bis -10° C, vorzugsweise 60 bis -10° C abgekühlt. In dieser Verfahrensstufe werden dabei alle Produkte kondensiert, die hoher als Cyanwasserstoff sieden. Cyanwasserstoff wird lediglich in dem Maße aus dem Pyrolysegas entfernt, wie es der Löslichkeit des Cyanwasserstoffs in den kondensierten Produkten entspricht. Setzt man die Verbindungen der Formel (I) zur Gewinnung von Cyanwasserstoff ein, so erhält man die N-Vinylsäureamide in der Verfahrensstufe, in der man das Pyrolysegas auf Temperaturen von 200 bis -10° C abkühlt. Vorzugsweise pyrolysiert man bei dem erfindungsgemäßen Verfahren Formamid.

Der nicht-kondensierte Cyanwasserstoff wird dann - ebenfalls unter vermindertem Druck, bei dem auch die Pyrolyse stattfindet - einer Chemiesorption unterworfen. Gegenüber der Pyrolyse tritt lediglich ein durch die Verfahrensführung bedingter Druckverlust auf. Der Druck in der Chemiesorptionszone des Verfahrens beträgt 5 bis 200, vorzugsweise 5 bis 45 mbar, wenn man den Cyanwasserstoff aus den Verbindungen der Formel I entwickelt und bei Herstellung des Cyanwasserstoffs aus Formamid 20 bis 60 mbar. Zur Chemiesorption des Cyanwasserstoffs und damit zur Herstellung von Umsetzungsprodukten des Cyanwasserstoffs verwendet man beispielsweise Natronlauge oder Kalilauge. In diesen Fällen erhält man die Natrium- bzw. Kalisalze der Cyanwasserstoffsäure. Ein anderes Sorbens für Cyanwasserstoff sind Carbonylverbindungen. Als Carbonylverbindungen eignen sich beispielsweise Aldehyde mit 1 bis 8 Kohlenstoffatomen, wie Formaldehyd, Acetaldehyd, Propionaldehyd, n-Butyraldehyd, Isobutyraldehyd und 2-Ethylhexanal. Als Carbonylverbindungen kommen auch Ketone, wie Aceton und Methylethylketon in Betracht. Die Chemiesorption des Cyanwasserstoffs mit Carbonylverbindungen erfolgt bei pH-Werten von 7 oder darüber. Vorzugsweise nimmt man die Chemiesorption mit Carbonylverbindungen im alkalischen pH-Bereich vor, weil unter diesen Bedingungen die Reaktion sehr schnell abläuft. Setzt man bei der Pyrolyse Formamid ein, so enthält das Spaltgas Ammoniak, der zusammen mit dem Cyanwasserstoff die Kondensatoren passiert und in der Chemiesorptionsstufe für einen ausreichend hohen pH-Wert sorgt. Man kann jedoch auch zusätzlich oder falls das Spaltgas wenig Ammoniak enthält, anorganische oder organische Basen in die Chemiesorptionsstufe einführen. Vorzugsweise

verwendet man zur Katalysierung der Reaktion zwischen Cyanwasserstoff und den Carbonylverbindungen üblicherweise tertiäre Amine, z. B. Triethylamin. Bei der Chemiesorption des Cyanwasserstoffs an Carbonylverbindungen kann man zusätzlich noch Amine verwenden, die am Stickstoff noch mindestens ein Wasserstoffatom tragen. In diesen Fällen erhält man als Umsetzungsprodukte des Cyanwasserstoffs in einer Streckersynthese Aminonitrile. Vorzugsweise verwendet man dabei als Amine Verbindungen der Formel

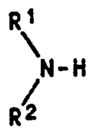 ,

in der $R^1$, $R^2$ = $C_1$- bis $C_8$-Alkyl oder H bedeuten. Cyanwasserstoff, Amine und Carbonylverbindungen werden vorzugsweise im Molverhältnis 1 : 1 : 1 bei der Chemiesorption eingesetzt.

Bei Aldehyden mit 1 bis 4 Kohlenstoffatome verläuft die Anlagerung des Cyanwasserstoffs bzw. die Anlagerung von Cyanwasserstoff und sekundären Aminen unter den Druckbedingungen der Pyrolyse mit einer solchen Geschwindigkeit, daß eine einstufige Chemiesorption ausreichend ist. Man kann jedoch die Chemiesorption in mehreren Verfahrensschritten, z.B. in zwei oder mehreren hintereinander geschalteten Kolonnen durchführen. Die Temperatur bei der Chemiesorption beträgt -20 bis +30, vorzugsweise -5 bis +15°C. Sofern die Reaktionsgeschwindigkeit bei der Chemiesorption mit Carbonylverbindungen abnimmt, arbeitet man üblicherweise mehrstufig, die Chemiesorption kann entweder unter Ausschluß eines Lösungsmittels oder in einem Lösungsmittel durchgeführt werden. Geeignete Lösungsmittel sind beispielsweise Wasser, Formamid oder Amine. Sofern Salze des Cyanwasserstoffs hergestellt werden, kann nicht auf den Einsatz eines Lösungsmittels bei der Chemiesorption verzichtet werden, weil die sich bei der Chemiesorption bildenden Salze in Lösung gehalten werden müssen.

Es ist überraschend, daß es unter den bei der Pyrolyse herrschenden Druckbedingungen - 5 bis 200 mbar - möglich ist, zwei so niedrig siedende Stoffe wie beispielsweise Cyanwasserstoff (Kp 27°C bei Normaldruck) und beispielsweise Acetaldehyd (Kp 21°C bei Normaldruck) bei einer Temperatur von +10°C fast quantitativ zu Milchsäurenitril umzusetzen. Dadurch ist es erstmals möglich, auf die Herstellung größerer Mengen an flüssigem Cyanwasserstoff (bei einer Produktion von 20 kmol pro Stunde sind dies immerhin 540 kg HCN pro Stunde) als Ausgangsmaterial für die Herstellung von Umsetzungsprodukten des Cyanwasserstoffs zu verzichten. Die Mengen an Cyanwasserstoff, die bei der Pyrolyse entstehen, werden in der Chemiesorptionsstufe, direkt zu den weitaus weniger gefährlichen Umsetzungsprodukten des Cyanwasserstoffs verarbeitet. Der Cyanwasserstoffgehalt dieser Umsetzungsprodukte liegt dabei in aller Regel unter 0,1 Gew.-%. Eine derartige Herstellung von Umsetzungsprodukten des Cyanwasserstoffs gewährleistet eine hohe Sicherheit auch bei Betriebsstörungen, weil beispielsweise bei den besonders gefürchteten Lekagen in der Druckapparatur die Cyanwasserstoffbildung sofort automatisch aufhört und damit jede weitere Cyanwasserstoffentwicklung gestoppt werden kann. Damit eröffnet sich die Möglichkeit, an fast beliebigen Standorten weit weniger problematische kleine Cyanwasserstoff-Folgeproduktionen, z. B. zur Herstellung von Acetoncyanhydrin bzw. Aminosäuren zu errichten. Weitere technische Vorteile des erfindungsgemäßen Verfahrens ergeben sich gegenüber dem Stand der Technik dadurch, daß sich bei einer Cyanhydrin- oder Aminonitril-Synthese eine $CO_2$-Wäsche gänzlich erübrigt. Ebenso ist es bei Einsatz von Formamid als Cyanwasserstoffbildner nicht mehr erforderlich, eine Ammoniakwäsche vorzunehmen.

Pyrolyse, Kondensation und Chemiesorption erfolgen, abgesehen vom Druckverlust, unter den gleichen Druckbedingungen, nämlich bei 5 bis 200 mbar. Die Pyrolyse und Chemiesorption werden immer kontinuierlich durchgeführt, in dem man den bei der Pyrolyse kontinuierlich entstehenden Cyanwasserstoff in dem Maße, in dem er gebildet wird, der Chemiesorption zuführt und von einer Kondensation des Cyanwasserstoffs absieht. Für die Chemiesorption ergeben sich mehrere Möglichkeiten. So kann der Cyanwasserstoff beispielsweise in eine überschüssige Menge des Sorptionsmittels, z. B. Aceton, eingeleitet werden oder man führt die Chemiesorption in einem Kreislauf durch, bei dem man das Sorptionsmittel, gegebenenfalls in einem Lösungsmittel, wie Formamid, über eine dazwischengeschaltete Kolonne umpumpt und dabei zu den im Kreis geführten Mischungen jeweils vor Eintritt in die Kolonne frisches Sorptionsmittel und gegebenenfalls frisches Lösungsmittel und andere Stoffe zudosiert und nach der Chemiesorption die Lösung in einem ebenfalls unter vermindertem Druck stehenden Behälter sammelt und daraus soviel ausschleust, wie vor der Kolonne zugegeben wird. Man erreicht dadurch einen stationären Zustand des Systems. In den Kreislauf ist ein Kühler integriert, mit dessen Hilfe die Temperatur bei der Chemiesorption geregelt wird.

**Beispiel 1**

Die Apparatur besteht aus einem Verdampfer, einem Pyrolyserohr, in dem als Katalysator eine Schüttung aus Magnesiumcarbonat und Calciumcarbonat auf α-Aluminiumoxid

angeordnet ist (Durchmesser des Katalysators 8 bis 14 mm), zwei Kondensatoren und einem Chemiesorptionskreis, in dem eine 45 %-ige wäßrige Glykolnitrillösung umgepumpt wird. Die Temperatur dieser Lösung wird auf +10° C eingestellt. Die Chemiesorption erfolgt in einer Kolonne, an deren Kopf man den aus der Pyrolyse stammenden Cyanwasserstoff und die wäßrige Glykolnitrillösung aufgibt und danach in einem Behälter sammelt, der mit einer Vakuumpumpe verbunden ist. Die Lösung gelangt dann im Chemiesorptionskreis über eine Umwälzpumpe und mehreren Dosierstellen zum Kopf der Kolonne.

Im Verdampfer werden bei einem Druck von 115 mbar stündlich 3,87 kg (86 Mol) Formamid kontinuierlich verdampft und die Dämpfe durch den auf eine Temperatur von 550° C erhitzten Katalysator geleitet. Die Pyrolysegase werden im ersten Kondensator auf eine Temperatur von 40 und in einem zweiten Kondensator auf eine Temperatur von +10° C abgekühlt. Hierbei werden nicht-umgesetztes Formamid und das bei der Umsetzung entstandene Wasser zurückgehalten. Die Spaltgase gelangen dann in den Chemiesorptionskreis, in dem man die wäßrige Glykolnitrillösung umpumpt und zu der man stündlich 7500 g einer 30 %-igen wäßrigen Formaldehydlösung einspeist. Der pH-Wert im Chemiesorptionskreis wird zwischen 8 und 9 gehalten. Der Gehalt an Cyanwasserstoff beträgt am unteren Ausgang der Kolonne weniger als 0,05 % und fällt im Verweilzeitbehälter unter die Nachweisgrenze ab. Inertgase, wie Luft, Kohlenmonoxid, Kohlendioxid und Wasserstoff passieren den Chemiesorptionskreis und gelangen auf die Druckseite der Vakuumpumpe. Aus dem Chemiesorptionskreislauf werden stündlich 9500 g einer 45 %-igen Glykolnitrilösung ausgeschleust und nach dem Druckausgleich mit der Atmosphäre auf Glykolnitril verarbeitet.

Aus den beiden Kühlern werden 180 g (6 Mol) pro Stunde an wiedereinsetzbarem Formamid ausgeschleust. Daraus errechnet sich bei einem Pyrolyseumsatz von 93 % eine Glykolnitrilausbeute von 93,8 %, bezogen auf umgesetztes Formamid.

**Beispiel 2**

Man benutzt die in Beispiel 1 beschriebene Apparatur, ersetzt jedoch im Chemiesorptionskreislauf die wäßrige Glykolnitrillösung durch eine 50 %-ige Milchsäurenitrillösung in Formamid. Zum Unterschied von Beispiel 1 wird der zweite Kondensator mit Sole beaufschlagt, so daß die Pyrolysegase darin auf eine Temperatur von -5 bis -10° C abgekühlt werden. In den Chemiesorptionskreislauf werden synchron 3212 g (73 Mol) Acetaldehyd und 5183 g Formamid pro Stunde zugeführt. Man erhält stündlich 10366 g einer 50 %-igen Lösung von Milchsäurenitril in Formamid entsprechend einer Ausbeute von 91,3 %, bezogen auf einen Formamidumsatz im Pyrolysereaktor von 93,0 %.

**Beispiel 3**

Man verwendet die in Beispiel 1 beschriebene Apparatur, pyrolysiert darin stündlich 2115 g (47 Mol) Formamid bei einer Temperatur von 480° C. Bei einem Umsatz von 88 % gelangen stündlich 1030 g Cyanwasserstoff in den Chemiesorptionskreis, in dem 5409 g (90 Mol) Ethylendiamin bei einer Temperatur von 25° C umgepumpt werden. Nach einer Stunde erhält man 6430 g einer Lösung von Ethylendiammoniumcyanid in überschüssigem Ethylendiamin.

**Patentansprüche**

1. Verfahren zur Herstellung von Umsetzungsprodukten des Cyanwasserstoffs durch Reaktion von Cyanwasserstoff mit Basen oder Carbonylverbindungen, dadurch gekennzeichnet, daß man durch Pyrolyse bei 250 bis 650° C an Feststoffen als Katalysatoren unter einem Druck von 5 bis 200 mbar hergestellten Cyanwasserstoff zusammen mit den anderen Pyrolyseprodukten auf eine Temperatur von 200 bis -10° C abkühlt, die höher als Cyanwasserstoff siedenden Produkte kondensiert, das Kondensat aus dem System ausschleust, den nichtkondensierten Cyanwasserstoff dann - ebenfalls unter vermindertem Druck, bei dem auch die Pyrolyse stattfindet - einer Chemiesorption mit Basen oder Carbonylverbindungen bei -20 bis +30° C zuführt und die dabei entstehenden Umsetzungsprodukte des Cyanwasserstoffs aus dem System ausschleust und auf Atmosphärendruck bringt.

2. Verfahren nach Anspruch 1, dadurch gekennzeichnet, daß man zur Herstellung des Cyanwasserstoffs Formamid und/oder N-Acylderivate des 1-Amino-1-cyanethans oder deren Substitutionsprodukte einsetzt.

3. Verfahren nach den Ansprüchen 1 und 2, dadurch gekennzeichnet, daß man zur Chemiesorption des Cyanwasserstoffs Natronlauge oder Kalilauge verwendet.

4. Verfahren nach den Ansprüchen 1 und 2, dadurch gekennzeichnet, daß man zur Chemiesorption des Cyanwasserstoffs Carbonylverbindungen verwendet.

5. Verfahren nach Anspruch 4, dadurch gekennzeichnet, daß man als Carbonylverbindung Aldehyde mit 1 bis 4 Kohlenstoffatomen verwendet.

6. Verfahren nach Anspruch 4, dadurch gekennzeichnet, daß man als Carbonylverbindung Ketone verwendet.

7. Verfahren nach Anspruch 4, dadurch

gekennzeichnet, daß man zusätzlich Amine der Formel

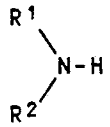

,

in der $R^1$, $R^2$ = $C_1$- bis $C_8$-Alkyl oder H bedeuten, einsetzt.

8. Verfahren nach den Ansprüchen 1 bis 7, dadurch gekennzeichnet, daß die Chemiesorption unter einem Druck von 5 bis 140 mbar durchgeführt wird.

## Claims

1. A process for the preparation of a reaction product of hydrogen cyanide by reacting the latter with a base or a carbonyl compound, wherein hydrogen cyanide prepared by pyrolysis at from 250 to 650°C over a solid as catalyst and under from 5 to 200 mbar is cooled to a temperature of from 200 to -10°C together with the other pyrolysis products, the products having a boiling point higher than that of hydrogen cyanide are condensed, the condensate is removed from the system, the uncondensed hydrogen cyanide is then fed - under the reduced pressure at which the pyrolysis is also carried out - to a chemisorption reaction with a base or with a carbonyl compound at from -20 to +30°C, and the resulting reaction product of hydrogen cyanide is removed from the system and brought to atmospheric pressure.

2. A process as claimed in claim 1, wherein formamide and/or an N-acyl derivative of 1-amino-1-cyanoethane or a substitution product thereof is used for the preparation of the hydrogen cyanide.

3. A process as claimed in claims 1 and 2, wherein sodium hydroxide solution or potassium hydroxide solution is used for the chemisorption of the hydrogen cyanide.

4. A process as claimed in claims 1 and 2, wherein a carbonyl compound is used for the chemisorption of the hydrogen cyanide.

5. A process as claimed in claim 4, wherein the carbonyl compound used is an aldehyde of 1 to 4 carbon atoms.

6. A process as claimed in claim 4, wherein the carbonyl compound used is a ketone.

7. A process as claimed in claim 4, wherein an amine of the formula

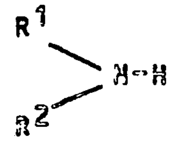

,

where $R^1$ and $R^2$ ar each $C_1$-$C_8$-alkyl or H, is additionally used.

8. A process as claimed in claims 1 to 7, wherein the chemisorption is carried out under from 5 to 140 mbar.

## Revendications

1. Procédé de préparation de produits de réaction de l'acide cyanhydrique par la réaction de l'acide cyanhydrique avec des bases ou des composés carbonylés, caractérisé en ce que l'acide cyanhydrique, préparé par pyrolyse entre 250 et 650°C sous une pression de 5 à 200 millibars en présence de matières solides comme catalyseurs, est refroidi avec les autres produits de la pyrolyse à une température comprise entre 200 et -10°C, les produits avec un point d'ébullition supérieur à celui de l'acide cyanhydrique sont condensés et le condensat est soutiré du système, puis l'acide cyanhydrique non condensé est soumis, à la même pression réduite, sous laquelle a lieu la pyrolyse et entre -20 et +30°C, à une chimisorption avec des bases ou des composés carbonylés et les produits de réaction de l'acide cyanhydrique formés sont soutirés du système et ramenés à la pression atmosphérique.

2. Procédé suivant la revendication 1, caractérisé en ce que l'on emploie, pour la préparation de l'acide cyanhydrique, du formamide et (ou) des dérivés N-acylés de l'amino-1 cyano-1 éthane ou des produits de substitution de ceux-ci.

3. Procédé suivant les revendications 1 et 2, caractérisé en ce que la chimisorption de l'acide cyanhydrique est réalisée avec de la lessive sodique ou de la lessive potassique.

4. Procédé suivant les revendications 1 et 2, caractérisé en ce que la chimisorption de l'acide cyanhydrique est réalisée avec des composés carbonylés.

5. Procédé suivant la revendication 4, caractérisé en ce que les composés carbonylés sont choisis parmi les aldéhydes en $C_1$ à $C_4$.

6. Procédé suivant la revendication 4, caractérisé en ce que les composés carbonylés sont choisis parmi les cétones.

7. Procédé suivant la revendication 4, caractérisé en ce que l'on utilise additionnellement des amines de de la formule

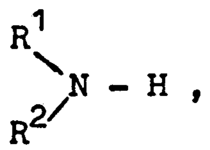

dans laquelle $R^1$ et $R^2$ désignent chacun un atome d'hydrogène ou un radical alkyle en $C_1$ à $C_8$.

8. Procédé suivant les revendications 1 à 7, caractérisé en ce que la chimisorption est réalisée sous une pression de 5 à 140 millibars.